# EUROPÄISCHE PATENTANMELDUNG

(11) **EP 1 350 492 A2**
(43) Veröffentlichungstag der Anmeldung: **08.10.2003**
(21) Anmeldenummer: 03007709.3
(22) Anmeldetag: 03.04.2003
(51) Int. Cl.: A61F 9/013

(54) **Einsatz für Augensaugring**

(30) Priorität: 04.04.2002 DE 10214917
(71) Anmelder: Gebauer GmbH, 75242 Neuhausen (DE)
(72) Erfinder: Gebauer, Detlev Paul, Dipl.-Ing., 75233 Tiefenbronn (DE)
(74) Vertreter: Leson, Thomas Johannes Alois, Dipl.-Ing.

(57) **Zusammenfassung**

Die Anmeldung betrifft einen Einsatz für einen Augensaugring, der bei ophthalmologischen Operationstechniken, wie z.B. in der refraktiven Chirurgie oder in der augenöffnenden invasiven Chirurgie zur Fixierung des Augapfels eingesetzt werden kann, und einen Augensaugring mit einem solchen Einsatz. Der ringförmige Einsatz (10) ist ein offenporiges Formteil. Als Werkstoff eignet sich beispielsweise Sinterglas, das aus miteinander versinterten 40 µm bis 100 µm großen Glaskugeln besteht. Der Augapfel (20) wird über einen Anschluss (5) des Augensaugrings durch den Einsatz (10) hindurch mit Unterdruck beaufschlagt, so dass er gegen eine Stirnfläche (13) des Einsatzes gesaugt wird. Der Augapfel (20) nimmt dadurch eine definierte Form ein, die wiederum einen bestimmten Augeninnendruck erzeugt. Der Einsatz (10) kann als Einwegartikel ausgeführt sein, der mittels einer Befestigung (7) in den Augensaugring eingesetzt und wieder von diesem getrennt werden kann.

## Beschreibung

Die Erfindung betrifft einen Einsatz für einen Augensaugring, der bei ophthalmologischen Operationstechniken zur Fixierung des Augapfels eingesetzt werden kann, und einen Augensaugring mit einem solchen Einsatz.

Bei verschiedenen ophthalmologischen Operationstechniken, wie z.B. in der refraktiven Chirurgie oder in der augenöffnenden invasiven Chirurgie muss das Auge fixiert werden, damit die Operation nicht durch eine ungewollte Augenbewegung des Patienten beeinträchtigt wird. Hierzu werden üblicherweise sogenannte Augensaugringe verwendet, die einen von einer Ringwand umgrenzten Hohlraum aufweisen, der den Augapfel aufnimmt. Über einen mit dem Hohlraum in Verbindung stehenden Anschluss wird ein Unterdruck angelegt, durch den der Augapfel in den Hohlraum gesogen und gegenüber dem Augensaugring fixiert wird.

Die DE 198 47 089 A1 offenbart einen solchen Augensaugring aus Edelstahl, der sich aufgrund seiner Ellipsenform insbesondere für LASIK-Operationen (LASIK: Laser-Assisted In Situ Keratomileusis) eignet, bei denen von dem Auge zunächst mittels eines Mikrokeratoms ein Hornhautlappen abgehobelt wird und die Hornhaut dann mit einem Laser behandelt wird, um Fehlsichtigkeiten zu korrigieren. Der Augensaugring weist auf der von dem Hohlraum abgewandten Seite eine Führungsnut zur Aufnahme des Mikrokeratoms auf und nimmt gegenüber dem Laser eine feste Lage ein.

Bei der LASIK-Operation ist es in der Regel erwünscht, dass der Augapfel durch den Unterdruck in den Hohlraum gesaugt wird und sich dabei geometrisch verformt, um einen größeren Hornhautlappen abhobeln zu können.

Allerdings schlägt sich die Verformung in einer Vergrößerung des Augeninnendrucks (Intraokulardruck IOP) nieder, der prinzipiell je nach Höhe und Zeit negative Auswirkungen auf das Auge haben kann. Unter Umständen kann es sogar zu einer Netzhautablösung kommen. Aus Gründen der Vorsicht sollte der Augeninnendruck daher nicht mehr als nötig erhöht werden.

Bei invasiven Operationen, wie z.B. bei Hornhauttransplantation oder bei Operationen des Grauen Stars, ist es dagegen gänzlich unerwünscht, wenn das Auge seine ursprüngliche Form ändert und der Augeninnendruck über dem normalen Druck liegt.

Die DE 38 38 253 A1 schlägt zu diesem Zweck einen Augensaugring aus Metall vor, in dessen von einer Ringwand umgebenen Hohlraum ein ringförmiger Metalleinsatz eingesetzt ist, der mit Schrauben gegen ein Herausfallen gesichert ist. Der Metalleinsatz hat eine an die Form des Augapfels angepasste Stirnfläche, die den Augapfel beim Anlegen des Unterdrucks stützt und in seiner ursprünglichen Form hält. Der Metalleinsatz stützt sich an der Innenwand des Hohlraums über Einzelsegmente ab, zwischen denen Kanäle verlaufen, durch die der Unterdruck zum Augapfel gelangt.

Der Erfindung liegt die Aufgabe zugrunde, eine praxisgerechte Alternative zu den aus dem Stand der Technik bekannten Augensaugringen beziehungsweise Ringeinsätzen zur Verfügung zu stellen, die eine definierte Einstellung des Augeninnendrucks erlaubt.

Eine erste Ausgestaltung der Erfindung sieht hierzu einen ringförmigen Einsatz für einen Augenring mit einer an die Form eines Augapfels angepassten Stirnfläche vor, der ein offenporiges Formteil ist.

Der Begriff "offenporig" steht für eine Porenstruktur, bei der die Poren des Formteils ein durchgängiges, nach außen hin offenes Netzwerk bilden. Wenn die Stirnfläche des ringförmigen Einsatz auf dem Augapfel sitzt, kann also ein Unterdruck, der auf der von der Stirnfläche des Einsatzes abgewandten Seite des Einsatzes anliegt, durch den Einsatz hindurch auf den Augapfel wirken, sodass der Augapfel gegen die Stirnfläche gesaugt wird. Der komplizierte Aufbau des aus der DE 38 38 253 A1 bekannten Einsatzes, bei dem der Unterdruck durch Kanäle zwischen den Einzelsegmenten geleitet werden muss, kann daher vermieden werden.

Dabei ist zu beachten, dass die an die Form des Augapfels angepasste Stirnfläche des Einsatzes nicht unbedingt so gestaltet sein muss, dass sich der Augapfel bei angelegtem Unterdruck überhaupt nicht oder nicht nennenswert verformt. Wie im einleitenden Teil beschrieben wurde, kann z.B. bei LASIK-Operationen eine gewisse Verformung des Augapfels durchaus erwünscht sein. Die Geometrie der Stirnfläche ist daher so angepasst, dass sich im Auge ein definierter Augeninnendruck bildet.

Da der Augapfel bei anliegendem Unterdruck durch die Stirnfläche des Einsatzes gestützt wird und eine definierte Form einnimmt, kann an den Augenring stets ein gleichbleibender, maximaler Unterdruck angelegt werden. Eine aufwendige Regelung des Unterdrucks, wie sie beispielsweise bei dem in der DE 19847089 A1 beschriebenen Augenring notwendig wäre, um ein zu starkes Hineinsaugen des Augapfels in den Hohlraum des Augenrings zu verhindern, ist daher nicht notwendig.

Die Stirnfläche des ringförmigen Einsatzes sollte vorzugsweise eine Rauheit aufweisen, die den Augapfel daran hindert, die Stirnfläche mit mehr als 10% seiner Fläche zu berühren. Auf diese Weise ergibt sich eine größere Angriffsfläche für den auf den Augapfel wirkenden Unterdruck und verbessert sich insgesamt die Ansaugwirkung.

Der ringförmige Einsatz lässt sich zum Beispiel durch Sintern herstellen, um ein aus miteinander versinterten Teilchen bestehendes Formteil zu erzielen. Zu diesem Zweck kann zum Beispiel eine Teilchenschüttung verwendet werden, die so aufgeheizt wird, dass die Teilchen nur im Oberflächenbereich aufschmelzen und sich an den Oberflächen miteinander verbinden. Es kann aber auch ein Gemisch aus mehreren Bestandteilen erhitzt und zu einem kompakten Körper gesintert werden, aus dem nachträglich ein Bestandteil herausgewaschen wird. Beide Verfahren sind dem Fachmann bekannt. Als Material für den durch Sintern hergestellten Einsatz eignen sich prinzipiell alle sinterfähigen Materialien wie Metall, Keramik, Kunststoff oder Glas.

Die miteinander versinterten Teilchen können prinzipiell eine Teilchengröße von 1 µm bis 1 mm haben, wobei sich jedoch eine Teilchengröße von 20 µm bis 250 µm und insbesondere 40 µm bis 100 µm als vorteilhaft herausgestellt hat.

Es ist empfehlenswert, dass die miteinander versinterten Teilchen kugelförmig oder zumindest kugelähnlich sind, da solche Teilchen zu einem besonders großen offenen Porenanteil führen. Außerdem hat diese Form den Vorteil, dass die Stirnfläche eine geeignete Rauheit aufweist, die es dem Augapfel ermöglicht, mit dem Einsatz in Punktkontakt zu bleiben. Dadurch erhöht sich wie gesagt die Angriffsfläche für den auf den Augapfel wirkenden Unterdruck.

Der Einsatz lässt sich zwar durch Sintern besonders kostengünstig herstellen, doch kommen anstelle von Sintern auch andere Fertigungstechniken in Frage, sofern sich damit offenporige Formteile herstellen lassen. Außerdem kann die Beschaffenheit der Stirnfläche und insbesondere ihre Rauheit auch durch Oberflächenbearbeitungstechniken wie Mikrostrukturverfahren, stereolithografische Verfahren oder laserunterstützte Verfahren eingestellt werden.

Eine weitere Ausgestaltung der Erfindung sieht einen Augenring mit einem von einer Ringwand umgrenzten Hohlraum vor, in den ein wie oben beschriebener ringförmiger Einsatz eingesetzt ist.

Der ringförmige Einsatz wird zwar bei der Operation durch das an der Stirnfläche anliegende Auge im Hohlraum des Augenrings festgehalten. Um jedoch die Handhabbarkeit zu verbessern und den Einsatz gegen ein unbeabsichtigtes Herausfallen aus dem Hohlraum zu sichern, sollte der Augenring eine Befestigung aufweisen, die den Einsatz hält. Die Befestigung kann beispielsweise ein Schnappverschluss sein.

Da sich der erfindungsgemäße Einsatz kostengünstig herstellen lässt, kann er im Gegensatz zu dem aus der DE 38 38 253 A1 bekannten Metalleinsatz als Einwegartikel eingesetzt werden, der nur für eine Operation genutzt und dann fortgeworfen wird. Reinigungs- und Sterilisationsprobleme entfallen daher. Außerdem lässt sich der Einsatz preisgünstig in vielen verschiedenen Geometrien anfertigen, sodass er abhängig von der jeweiligen Größe des Auges, der gewünschten Augenverformung und dem angestrebten Augeninnendruck bei jeder Operation individuell ausgewählt werden kann.

Wenn der Augenring aus einem langzeitbeständigen Material wie Metall besteht, sollte die oben angesprochene Befestigung lösbar sein, um den Einsatz von dem Augensaugring trennen und gegen einen neuen Einwegeinsatz austauschen zu können.

Allerdings kann der Augensaugring auch zusammen mit dem Einsatz als Einwegartikel ausgeführt sein. Der Augensaugring besteht in diesem Fall vorzugsweise aus Kunststoff, wobei der Einsatz entweder mit Hilfe eines Befestigungsmittels wie den angesprochenen Schnappverschluss befestigt wird oder aber durch eine Klebung oder durch Formschluss dauerhaft mit dem Augenring verbunden wird.

Abgesehen davon kann der Augensaugring einen mit dem Hohlraum in Verbindung stehenden Anschluss aufweisen, der über eine Leitung mit einer Vakuumpumpe verbunden wird, um den Unterdruck anzulegen. In diesem Fall ist es besonders vorteilhaft, wenn der offenporige Einsatz das zum Hohlraum hin offene Ende des Anschlusses bedeckt. Der Einsatz wirkt dann als ein Filter, der Augengewebe (z.B. die Sklera), das sich während der Operation löst, zurückhält und daran hindert, in den Anschluss gesogen zu werden und diesen zu verstopfen. Dadurch wird vermieden, dass ein Sensor, der die Absaugleistung der Vakuumpumpe erfasst, wegen des verstopften Anschlusses irrtümlich einen hohen Unterdruck erkennt, obwohl der Unterdruck in dem Augenring in Wahrheit unzureichend ist. Die Operation wird also für den Patienten sicherer.

Der Augensaugring kann außerdem auf der von dem Hohlraum abgewandten Seite eine Einrichtung zur Aufnahme von Operationshilfsmitteln aufweisen, etwa eine Führungsnut für ein Mikrokeratom, mit dem bei LASIK-Operationen ein Hornhautlappen abgehobelt wird.

Mit dem oben beschriebenen Augensaugring lässt sich wie gesagt beim Fixieren des Augapfels ein definierter Augeninnendruck einstellen. Und zwar kann trotz Unterdruckbeaufschlagung eine Veränderung des Augeninnendrucks vermieden werden, wenn die Stirnfläche des von dem Augensaugring gehaltenen Einsatzes die gleiche Krümmung wie der Augapfel aufweist. Andererseits kann der Augeninnendruck auch bei Bedarf definiert erhöht werden, wenn die Stirnfläche so gestaltet ist, dass ihre Krümmung größer als die des Augapfels ist.

Die Erfindung wird nun näher anhand eines Ausführungsbeispiels beschrieben. Dabei wird auf die beigefügten Zeichnungen Bezug genommen, die Folgendes zeigen:
Fig. 1 eine schematische Schnittansicht eines Augensaugrings mit einem erfindungsgemäßen Einsatz, der auf einem Augapfel sitzt;
Fig. 2 eine vergrößerte Teilansicht von Fig. 1 ohne den Augapfel;
Fig. 3 eine Perspektivansicht des Augensaugrings von der von dem Einsatz abgewandten Seite aus gesehen; und
Fig. 4 eine genauere Schnittansicht des Einsatzes.

Die Figuren zeigen einen Augensaugring für eine LASIK-Operation.

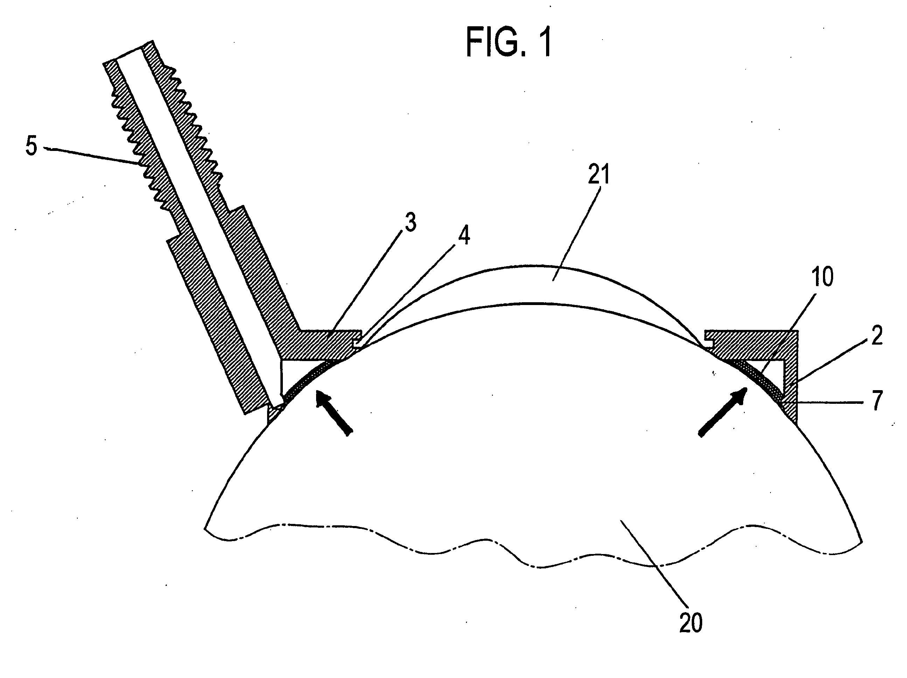

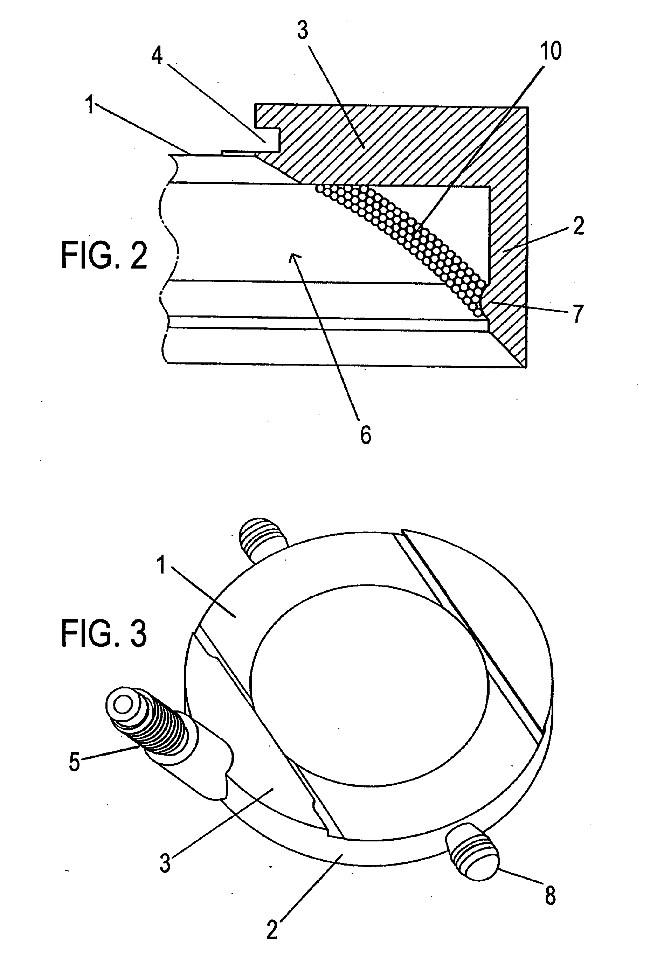

Wie in den Schnittansichten der Figuren 1 und 2 dargestellt ist, setzt sich der aus Edelstahl bestehende Grundkörper des Augensaugrings aus einer Ringplatte 1 zusammen, von deren Außenumfang in Normalenrichtung eine Ringwand 2 abgeht, sodass sich im Querschnitt ein rechtwinkliges Profil ergibt. Die Ringwand 2 umgrenzt einen Hohlraum 6, der bei der LASIK-Operation den Augapfel 20 des Patienten aufnimmt.

Wie insbesondere in der Perspektivansicht von Fig. 3 zu erkennen ist, weist die Ringplatte 1 an auf ihrer von dem Hohlraum 6 abgewandten Oberseite zwei parallel verlaufende Erhebungen 3 auf, die in ihrer zur Mitte der Ringplatte 1 weisenden Seitenfläche jeweils eine Führungsnut 4 aufweisen. Die Führungsnuten 4 bilden zusammen mit dem Bereich der Ringplatte 1 zwischen den beiden Erhebungen 3 eine Führung für ein (nicht gezeigtes) Mikrokeratom. Das Mikrokeratom wird während der LASIK-Operation in die Führungsnuten 4 eingeschoben, um von der Hornhaut 21 des Augapfels 20, die von dem Hohlraum 6 aus über den Rand der Ringplatte 1 ragt, einen Lappen abzuhobeln.

In der Einschubrichtung des Mikrokeratoms weist der Augensaugring außerdem zwei von der Ringwand 2 an entgegengesetzten Stellen nach außen weisende Zapfenlager 8 auf, mit deren Hilfe der Augensaugring mit der Operationsapparatur verbunden werden kann.

Der Augensaugring hat einen hohlzylinderförmigen Anschluss 5, der von der Oberseite der Ringplatte 1 schräg von außen in die Ringwand 2 mündet und mit dem Hohlraum 6 in Verbindung steht. Der Anschluss 5 ist an seinem von der Ringwand 2 fernen Ende mit einem Gewinde versehen, an das während der LASIK-Operation eine (nicht gezeigte) Leitung geschraubt wird. Diese Leitung ist an eine Vakuumpumpe angeschlossen, die aus dem Hohlraum 6 Luft absaugt, um darin einen Unterdruck zu erzeugen.

Wie in Figuren 1 und 2 dargestellt ist, ist in den Hohlraum 6 des Augensaugrings ein ringförmiger Einsatz 10 eingesetzt. Der ringförmige Einsatz 10 wird von einem Schnappverschluss gegen Herausfallen gesichert. Der Schnappverschluss wird von einem Vorsprung 7 gebildet, der an der Innenseite des von der Ringplatte 1 fernen Endes der Ringwand 2 entlang läuft.

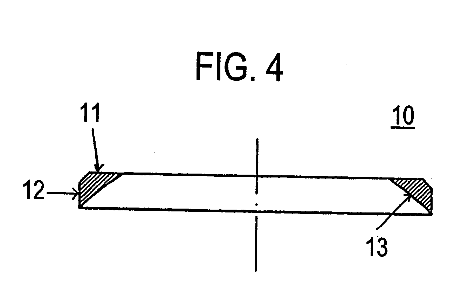

Die genaue Form des Einsatzes 10 ist in Fig. 4 dargestellt. Die mit den Innenseiten des Hohlraums 6 in Berührung kommende Oberseite 11 und die dazu rechtwinklige Mantelfläche 12 des Einsatzes 10 sind so ausgeführt, dass sie bündig an der Innenseite der Ringwand 2 und der Unterseite der Ringplatte 1 des Augensaugrings anliegen. Die Kante zwischen der Oberseite 11 und der Mantelfläche 12 des Einsatzes 10 ist abgeschrägt, um das Einsetzen in den Hohlraum 6 zu erleichtern. An die Oberseite 11 und die Mantelfläche 12 des Einsatzes 10 schließt sich die Stirnfläche 13 an, sodass der Einsatz 10 im Halbschnitt ungefähr ein dreieckiges bzw. unter Berücksichtigung der Abschrägung ein trapezförmiges Profil hat.

Die Stirnfläche 13 des Einsatzes 10, die während der Operation auf dem Augapfel 20 sitzt, weist mit einem Krümmungsradius von knapp 13 mm eine etwas stärkere Krümmung als der Augapfel 20 auf und definiert an ihrem oberen Ende ein Ringloch mit einem Durchmesser von ungefähr 14 mm und an ihrem unteren Ende ein Ringloch mit einem Durchmesser von ungefähr 18 mm. Der Einsatz 10 ist insgesamt weniger als 2 mm hoch. Durch die oben beschriebene Geometrie des Einsatzes 10 kommt es während der Operation zu einer leichten Ausbeulung des Augapfels 20 (durch die Pfeile in Fig. 1 markiert). Dadurch erhöht sich der Augeninnendruck um ein definiertes Maß und treibt die Hornhaut 21 etwas weiter aus dem Hohlraum 6 heraus, was das Abhobeln des Hornhautlappens erleichtert.

Der Einsatz 10 besteht bei diesem Ausführungsbeispiel aus Sinterglas, in dem zwischen 40 µm und 100 µm große Glaskugeln zu einem offenporigen Formteil versintert sind. Sinterglas ist in erster Linie als Filtermedium oder als Zuchthilfe für Mikrobakterien bekannt. Aufgrund seines großen offenen Porenanteils, der Möglichkeit zur endformnahen Fertigung und der geringen Material- und Herstellungskosten eignet sich Sinterglas jedoch auch als Einsatz für Augensaugringe.

Da der Einsatz 10 nur wenige Millimeter breit und hoch ist, besteht der Einsatz 10 aus nur wenigen Lagen Glaskugeln und reihen sich zwischen dem oberen und unteren Ende der Stirnfläche 13 nur einige Dutzend Glaskugeln aneinander. Der Einsatz 10 hat dennoch eine für den Einsatzzweck ausreichende mechanische Festigkeit.

Die Verarbeitung von Glaskugeln definierter Größe zu Sinterglas ist Stand der Technik und wird auf dem Markt von einer Reihe von Fertigungsbetrieben angeboten. Die Fertigungsverfahren decken sich in der Regel insoweit, als dass die Glaskugeln in ein Werkzeug geschüttet und bei einer Temperatur gesintert werden, bei der die Kugeln nur leicht an den Oberflächen anbacken, und dass das erzielte Formteil schließlich spanabhebend zu einem endformnahen Rohling bearbeitet wird. Das Formteil lässt sich dabei problemlos in verschiedenen Geometrien anfertigen, mit denen sich während der Operation definierte Augeninnendrücke erzielen lassen.

Der Einsatz 10 stellt bei diesem Ausführungsbeispiel einen Einwegartikel dar, der nur für eine Operation genutzt und dann fortgeworfen wird, während der aus Edelmetall bestehende Augenring mehrfach zum Einsatz kommt. Der Augenring weist aus diesem Grund den Schnappverschluss 7 auf, der es ermöglicht, den Einsatz 10 nach der Operation von dem Augensaugring zu trennen und gegen einen neuen Einsatz auszutauschen.

Bei dem obigen Ausführungsbeispiel wurden im Einsatz 10 für das Sinterglas Glaskugeln mit einer Größe zwischen 40 µm und 100 µm verwendet, da sich dieser Wertebereich als besonders günstiger Kompromiss zwischen Fertigungs- und Produkteigenschaften erwiesen hat. Andere Größen sind jedoch prinzipiell auch möglich. Als fertigungstechnische Obergrenze wird eine Größe von 1 mm angesehen, da in diesem Fall zwischen dem oberen und unteren Ende der Stirnfläche 13 des Einsatzes 10 nur etwa ein halbes Dutzend Glaskugeln aneinander gereiht werden könnten. Außerdem wären die Zwischenräume bzw. Poren zwischen den einzelnen Glaskugeln so groß, dass eventuell während der Operation abgelöstes Augengewebe nur zum Teil zurückgehalten werden könnte und den Unterdruckanschluss 5 verstopfen würde. Als Untergrenze wird eine Größe von 1 µm angesehen, da dann nicht mehr ausgeschlossen werden kann, dass sich die Poren beim Versintern so weit schließen, dass das Formteil nicht mehr offenporig ist. Außerdem nimmt Oberflächenrauheit bei einer Größe von weniger als 1 µm so stark ab, dass der Augapfel im Großen und Ganzen flächig an der Stirnfläche 13 anliegen und die Angriffsfläche für den Unterdruck zu stark abnehmen würde. In einem Größenbereich von 20 µm bis 250 µm lassen sich in der Regel noch zufriedenstellende Ergebnisse erzielen.

Der erfindungsgemäße Einsatz 10 muss im Übrigen nicht unbedingt aus Sinterglas bestehen. Andere Materialien, etwa Sintermetalle, sind zwar teurer, können jedoch auch mit etwas höherer Maßgenauigkeit hergestellt werden.

Der Augensaugring und der Einsatz 10 müssen nicht unbedingt kreisförmig sein. Eine ovale oder mehreckige Außenkontur ist ebenfalls möglich.

Der Einsatzbereich des Augenrings beschränkt sich nicht auf LASIK-Operationen. Der Augenring kann, entsprechend modifiziert, auch bei anderen refraktiven Operationen oder bei augenöffnenden invasiven Operationen wie Hornhautoperationen oder Operationen des Grauen Stars eingesetzt werden. Bei den augenöffnenden Operationen ist zu beachten, dass die Krümmung der Stirnfläche 13 des Einsatzes 10 der des Augapfels 20 entspricht, um eine Verformung des Augapfels durch den Unterdruck und damit eine Erhöhung des Augeninnendrucks weitestgehend zu vermeiden.

Wie bereits angesprochen wurde, kann der Augenring auch als Ganzes in Form eines Einwegartikels ausgeführt werden. Im beschriebenen Ausführungsbeispiel ist dies jedoch problematisch, da an die Maßgenauigkeit der Führungsnuten 4 des Augenrings, die beim Abhobeln des Hornhautlappens das Mikrokeratom führen sollen, verhältnismäßig hohe Ansprüche gestellt werden, die nur schwer von einem Kunststoffformteil erfüllt werden können. Die Fertigung des Augenrings aus Kunststoff kann jedoch insbesondere dann in Betracht gezogen werden, wenn der Augenring keine Operationsmittel wie das Mikrokeratom aufnehmen muss, wie z.B. bei Operationen, bei denen das Auge direkt mit Laserlicht behandelt wird.

Die Anmeldung betrifft einen Einsatz für einen Augensaugring, der bei ophthalmologischen Operationstechniken, wie z.B. in der refraktiven Chirurgie oder in der augenöffnenden invasiven Chirurgie zur Fixierung des Augapfels eingesetzt werden kann, und einen Augensaugring mit einem solchen Einsatz. Der ringförmige Einsatz (10) ist ein offenporiges Formteil. Als Werkstoff- eignet sich beispielsweise Sinterglas, das aus miteinander versinterten 40 µm bis 100 µm großen Glaskugeln besteht. Der Augapfel (20) wird über einen Anschluss (5) des Augensaugrings durch den Einsatz (10) hindurch mit Unterdruck beaufschlagt, so dass er gegen eine Stirnfläche (13) des Einsatzes gesaugt wird. Der Augapfel (20) nimmt dadurch eine definierte Form ein, die wiederum einen bestimmten Augeninnendruck erzeugt. Der Einsatz (10) kann als Einwegartikel ausgeführt sein, der mittels einer Befestigung (7) in den Augensaugring eingesetzt und wieder von diesem getrennt werden kann.

## Patentansprüche

1. Ringförmiger Einsatz (10) für einen Augensaugring, der eine an die Form eines Augapfels (20) angepasste Stirnfläche (13) hat, **dadurch gekennzeichnet, dass** der Einsatz (10) ein offenporiges Formteil ist.

2. Ringförmiger Einsatz nach Anspruch 1, dessen Stirnfläche (13) eine Rauheit aufweist, die den Augapfel (20) daran hindert, die Stirnfläche mit mehr als 10% seiner Fläche zu berühren.

3. Ringförmiger Einsatz nach Anspruch 1 oder 2, der aus miteinander versinterten Teilchen besteht.

4. Ringförmiger Einsatz nach Anspruch 3, bei dem die miteinander versinterten Teilchen eine Teilchengröße von 1 µm bis 1 mm haben.

5. Ringförmiger Einsatz nach Anspruch 3, bei der die miteinander versinterten Teilchen eine Teilchengröße von 20 µm bis 250 µm haben.

6. Ringförmiger Einsatz nach Anspruch 3, bei der die miteinander versinterten Teilchen eine Teilchengröße von 40 µm bis 100 µm haben.

7. Ringförmiger Einsatz nach Anspruch 3, bei der die miteinander versinterten Teilchen kugelförmig sind.

8. Augensaugring mit einem von einer Ringwand (2) umgrenzten Hohlraum (6), in den ein ringförmiger Einsatz (10) gemäß einem der Ansprüche 1 bis 7 eingesetzt ist.

9. Augensaugring nach Anspruch 8, mit einer Befestigung (7) für den Einsatz (10), um den Einsatz gegen ein Herausfallen aus dem Hohlraum (6) zu sichern.

10. Augensaugring nach Anspruch 9, bei dem die Befestigung (7) ein Schnappverschluss ist.

11. Augensaugring nach einem der Ansprüche 9 oder 10, der aus Metall besteht und bei dem die Befestigung (7) gelöst werden kann, um den Einsatz (10) von dem Augensaugring zu trennen.

12. Augensaugring nach einem der Ansprüche 8 bis 10, der aus Kunststoff besteht.

13. Augensaugring nach Anspruch 8, der einen mit dem Hohlraum (6) in Verbindung stehenden Anschluss (5) zum Anlegen von Unterdruck aufweist.

14. Augensaugring nach Anspruch 13, bei dem der Einsatz (10) ein zum Hohlraum (6) hin offenes Ende des Anschlusses (5) bedeckt.

15. Augensaugring nach Anspruch 8, der auf der von dem Hohlraum (6) abgewandten Seite eine Einrichtung (4) zur Aufnahme von Operationshilfsmitteln aufweist.

16. Verwendung eines Augensaugrings gemäß einem der Ansprüche 8 bis 15 zur Fixierung eines Augapfels (20), wobei die Stirnfläche (13) des Einsatzes (10) die gleiche Krümmung wie der Augapfel (20) aufweist, damit sich der Augeninnendruck nicht verändert.

17. Verwendung eines Augensaugrings gemäß einem der Ansprüche 8 bis 15 zur Fixierung eines Augapfels (20), wobei die Stirnfläche (13) des Einsatzes (10) eine größere Krümmung als der Augapfel (20) aufweist, damit sich der Augeninnendruck definiert erhöht.
